# EUROPÄISCHE PATENTSCHRIFT

(11) **EP 4 046 671 B1**
(45) Veröffentlichungstag und Bekanntmachung des Hinweises auf die Patenterteilung: **25.09.2024**
(21) Anmeldenummer: 22157246.4
(22) Anmeldetag: 17.02.2022
(51) Int. Cl.: A61M 3/02

(54) **SPÜLANORDNUNG**
FLUSHING ARRANGEMENT
AGENCEMENT DE RINÇAGE

(30) Priorität: 19.02.2021 DE 202021100846 U
(43) Veröffentlichungstag der Anmeldung: 24.08.2022
(73) Patentinhaber: UTK Solution GmbH, 58507 Lüdenscheid (DE)
(72) Erfinder: Thiessies, Olaf, 58513 Lüdenscheid (DE)
(74) Vertreter: Patentanwälte Dörner & Kötter PartG mbB

(56) Entgegenhaltungen:
- CN-A- 111 134 953
- US-A1- 2005 165 431
- US-A1- 2008 146 991

## Beschreibung

Die Erfindung betrifft eine Spülanordnung für Spülsysteme zur Reinigung von Operationswunden mittels Spülflüssigkeit umfassend ein Außenrohr, in dem ein Innenrohr angeordnet ist, wobei zwischen Außenrohr und Innenrohr mindestens abschnittsweise ein Ringspalt ausgebildet ist, und an dem freien Ende des Außenrohrs ein Aufsatz vorgesehen ist, der auf seinem Umfang Borsten aufweist.

Spülsysteme (sog. Lavage-Systeme) der hier betrachteten Art dienen zur Reinigung von Operationswunden, um nekrotisches Gewebe, Bakterien und Fremdmaterial aus der Wunde zu spülen. Insbesondere bei Operationen am Knochen, wie zum Beispiel beim Einsetzen von künstlichen Gelenken, werden solche Spülsysteme eingesetzt, um Rückstände, die bei der Operation entstehen, auszuspülen. Dadurch sollen Komplikationen nach der Operation vermieden und die Heilung beschleunigt werden, zumal Rückstände die Haltbarkeit des künstlichen Gelenks beeinträchtigen und zu Infektionen im Bereich des Knochens führen können. Die Spülung soll dabei gründlich aber gleichzeitig schonend erfolgen.

Durch die Spülsysteme wird ein laminarer oder pulsierender Strahl erzeugt, der durch ein Rohr zu einer Düse gelangt und durch die Düse aus dem System austritt. Bei der Flüssigkeit handelt es sich überwiegend um sterile Kochsalzlösung oder eine wässrige Lösung, die auch pharmazeutisch wirksame Substanzen enthalten kann. Die mit den Rückständen kontaminierte Flüssigkeit wird durch besondere Sauger über einen zweiten Kanal abgesaugt. Die Sauger haben eine trichterförmige Gestalt und können Bestandteil des Spülsystems sein. Die Sauger können aber auch in Form einer separaten Vorrichtung ausgebildet sein.

Ein wesentliches Kriterium für die Qualität der Spülung neben dem Spülvolumen ist der Druck, mit dem die Flüssigkeit auf den Knochen, die Spongiosa oder das Gewebe trifft. Ist der Strahl zu fest, kann dies zu einer Schädigung des Knochens o. dgl. führen. Schädigende Bestandteile können dann statt ausgespült tiefer in den Knochen gedrückt werden.

Bei den Spülsystemen finden unterschiedliche Gestaltungen des Spül- bzw. Absaugrohrs Anwendung. So sind Gestaltungen bekannt, bei denen in dem Absaugrohr koaxial das Spülrohr angeordnet ist (vgl. bspw. DE 10 2012 000 392 A1). Der Spülstrahl tritt folglich in der Mitte aus der Düse aus. Die Spülflüssigkeit wird dabei über den um das Spülrohr herum ausgebildeten Ringspalt aufgesaugt. Aufgrund der geringen Abmessungen weist diese Lösung aber den Nachteil auf, dass der Ringspalt beim Absaugen größerer Mengen an kontaminierter Flüssigkeit verstopfen kann.

Zur Vermeidung dieses Nachteils sind Gestaltungen bekannt, bei denen in dem Spülrohr koaxial das Absaugrohr angeordnet ist (vgl. bspw. DE 20 2010 011 728 U1). Die Düse am Spülrohr umgibt folglich das Absaugrohr. Das Absaugen erfolgt somit zentral in der Mitte des Spül- und Absaugrohrs, wodurch der vorgenannte Nachteil vermieden ist.

Zum Spülen von röhrenförmigen Hohlräumen von Organen (sog. Lumen) finden Spülsysteme Anwendung, bei denen vor der Düse eine Prallplatte angeordnet ist, die die Spülflüssigkeit nach dem Austritt aus der Düse rechtwinklig umlenkt, so dass die vom Knochen bzw. Spongiosa gebildete Wandung gespült werden kann. Der Strahl trifft daher ebenfalls senkrecht auf die zu spülende Knochen-oder Gewebefläche. Das Absaugen erfolgt auch hier über einen Ringspalt, der die Düse umgibt.

Zur Verbesserung der Reinigung der Lumen finden zudem Spülsysteme Anwendung, bei denen auf dem Umfang Borsten angeordnet sind. Diese unterstützen das Spülen, indem die Borsten nach Art einer Bürste die Wandung des jeweiligen Hohlraums mechanisch bearbeiten und dabei das nekrotisches Gewebe, Bakterien oder Fremdmaterial aus der Wunde lösen. Sie finden beispielsweise bei der Reinigung von Knochenzement oder Resten von speziellen medizinischen Klebern Anwendung, welche häufig bei dem Einsetzen von Hüftprothesen verwendet werden.

Bei den bekannten Spülanordnungen haben die Borsten eine zur Mittelachse linienförmige und parallele Anordnung der Borstenreihen (vgl. bspw. US 2008/0146991 A1). Dies macht es notwendig, das eingeführte Rohr sowohl axial als auch rotatorisch in dem Lumen zu bewegen, um alle Bereiche der zu reinigenden Oberfläche zu erreichen. Da der zu reinigende Bereich für den Anwender nicht einsehbar ist, kann das Reinigungsergebnis nicht kontrolliert werden.

Vor diesem Hintergrund liegt der Erfindung die Aufgabe zugrunde, ein Spülsystem zu schaffen, bei dem lediglich eine axiale Bewegung erforderlich ist, um alle Stellen der zu reinigenden Oberfläche zu erreichen. Gemäß der Erfindung wird diese Aufgabe mit den Merkmalen des Patentanspruchs 1 gelöst.

Mit der Erfindung ist ein Spülsystem geschaffen, bei dem lediglich eine axiale Bewegung erforderlich ist, um alle Stellen der zu reinigenden Oberfläche zu erreichen. Da die Borsten nach Art einer Helix auf dem Umfang angeordnet sind, folgen sie einer Spirale um das Rohr. Durch diese Anordnung wird das Problem der zusätzlichen Rotationsbewegung beseitigt. Es ist nur ein axiales Einschieben der Spülanordnung erforderlich. Durch die Anordnung der Borsten wird bei dem Einschieben jeder Bereich erreicht und mechanisch gebürstet.

In Weiterbildung der Erfindung sind die Borsten konisch ausgeführt. Durch die konische Ausführung der Borsten wird eine Biegelinie erreicht, um eine ausreichende Steifigkeit der Borsten bei gleichzeitiger möglichst schonender Reinigung an der Oberfläche zu erzielen.

In anderer Weiterbildung der Erfindung ist der Aufsatz kappenartig ausgebildet und vorzugsweise außen abgerundet ausgeführt. Dadurch sind die scharfen Kanten des Rohres abgedeckt und das Knochengewebe beim Einschieben in Lumina geschont.

Andere Weiterbildungen und Ausgestaltungen der Erfindung sind in den übrigen Unteransprüchen angegeben. Ausführungsbeispiele der Erfindung sind in den Zeichnungen dargestellt und werden nachfolgend im Einzelnen beschrieben. Es zeigen:
- Figur 1: die Ansicht einer nicht erfindungsgemäßen Spülanordnung;
- Figur 2: die stirnseitige Ansicht der in Figur 1 dargestellte Spülanordnung;
- Figur 3: den Schnitt entlang der Linie III - III in Figur 2;
- Figur 4: den in Figur 3 dargestellten Längsschnitt durch das Adapterstück in vergrößertem Maßstab;
- Figur 5: den in Figur 3 dargestellten Längsschnitt durch den Aufsatz in vergrößertem Maßstab;
- Figur 6: die Einzelheit "Z" aus Figur 5 in vergrößertem Maßstab;
- Figur 7: die stirnseitige Ansicht der in Figur 1 dargestellte Spülanordnung in vergrößerter Darstellung;
- Figur 8: die Ansicht einer Spülanordnung in anderer Ausbildung, die nicht der beanspruchten Erfindung entspricht;
- Figur 9: die stirnseitige Ansicht der in Figur 8 dargestellte Spülanordnung;
- Figur 10: den Schnitt entlang der Linie IX - IX in Figur 9;
- Figur 11: den in Figur 10 dargestellten Längsschnitt durch den Aufsatz in vergrößertem Maßstab;
- Figur 12: die Ansicht einer Spülanordnung in einer weiteren Ausbildung (erfindungsgemäße Spülanordnung wie beansprucht);
- Figur 13: die stirnseitige Ansicht der in Figur 12 dargestellte Spülanordnung;
- Figur 14: den Schnitt entlang der Linie XIII - XIII in Figur 13;
- Figur 15: die in Figur 12 dargestellte Ansicht des Aufsatzes in vergrößertem Maßstab;
- Figur 16: den Schnitt entlang der Linie XIV - XIV in Figur 14 in vergrößertem Maßstab;

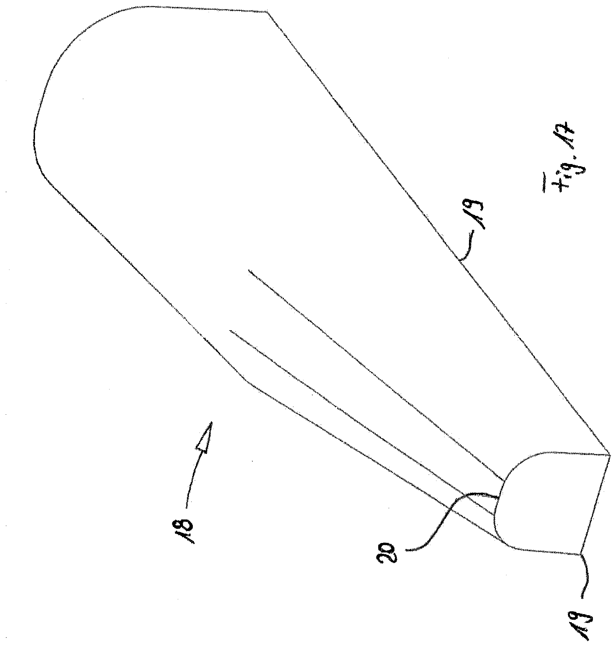

- Figur 17: die vergrößerte dreidimensionale Darstellung einer Borste;
- Figur 18: die vergrößerte dreidimensionale Darstellung des Aufsatzes.

Die in den Ausführungsbeispielen dargestellten Spülanordnungen sind für Spülsysteme zur Reinigung von Operationswunden mittels Spülflüssigkeit vorgesehen. Jede Spülanordnung umfasst ein Außenrohr 1, in dem ein Innenrohr 2 angeordnet ist. Zwischen dem Außenrohr 1 und Innenrohr 2 ist abschnittsweise ein Ringspalt 3 ausgebildet. Je nach Ausführung der Spülanordnung kann sich der Ringspalt auch über die gesamte Länge des Außenrohrs 1 und des Innenrohrs 2 erstrecken.

An einem Ende des Außenrohrs 1 ist ein Adapterstück 4 zum Anschluss an eine nicht dargestellte Antriebs- und Betätigungseinheit angeordnet, wie sie aus dem Stand der Technik bekannt ist. An dem dem Adapterstück 4 abgewandten Ende des Außenrohrs 1 ist ein Aufsatz 5 vorgesehen.

Das Adapterstück 4 übergreift flüssigkeitsdicht mit seinem dem Aufsatz 5 zugewandten Ende das eine Ende des Außenrohres 1. Die Gestaltung des Adapterstücks 4 erweitert sich in Richtung der dem Aufsatz 5 abgewandten Seite. In dem Adapterstück ist ein Einsatz 6 angeordnet, der eine Pumpenaufnahme 7 und ein Rohrabschnitt 8 aufweist. Von dem Rohrabschnitt 8 ist auf der einen Seite das dem Adapterstück 4 abgewandten Ende des Innenrohrs 2 aufgenommen. Die andere Seite des Rohrabschnitts 8 ist zum Anschluss eines weiteren Schlauchs vorgesehen.

In die Pumpenaufnahme 7 ragt in montiertem Zustand der Spülanordnung eine stabile Pumpe, die mittels eines O-Rings gegen die Pumpenaufnahme 7 abgedichtet ist und die die Spülflüssigkeit zuführt; an den Rohrabschnitt 8 wird zur Benutzung der Spülanordnung ein Saugschlauch angeschlossen, der die kontaminierte Spülflüssigkeit abführt. Aus dieser Anordnung folgt, die Zufuhr der Spülflüssigkeit zum Aufsatz 5 durch den Ringspalt 3 erfolgt und das Abführen der kontaminierten Spülflüssigkeit durch das Innenrohr 2 erfolgt.

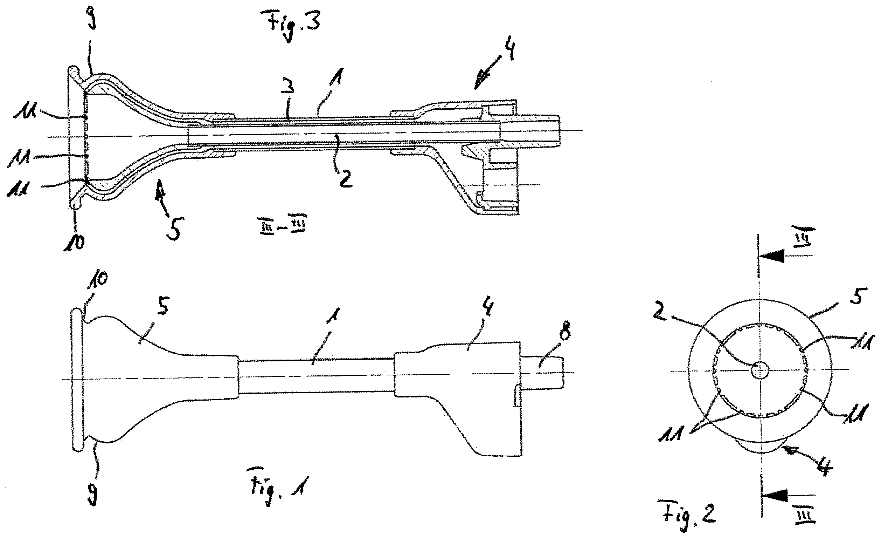

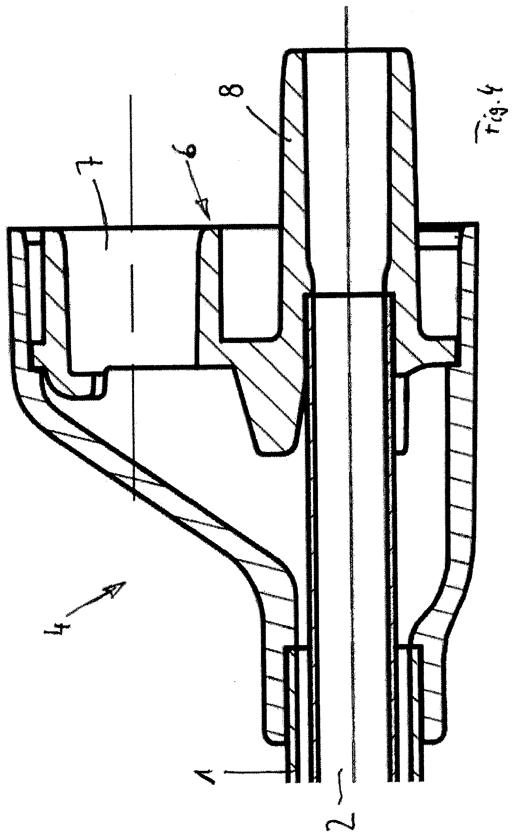

Der Aufsatz 5 ist unterschiedlich gestaltet, wie anhand der Figuren ersichtlich ist. Im (nicht erfindungsgemäßen) Ausführungsbeispiel nach den Figuren 1 bis 6 ist der Aufsatz 5 im Wesentlichen glockenförmig. In seinem dem Adapterstück 4 abgewandten Bereich weist der Aufsatz 5 eine Einschnürung 9 auf, an die sich ein trichterförmiger Abschnitt 10 anschließt, welcher das freie Ende des Aufsatzes 5 bildet. In dem dem Abschnitt 10 zugewandten Ende der Einschnürung 9 und damit unmittelbar benachbart zum Abschnitt 10 sind in dem Aufsatz 5 benachbart zueinander Austrittsöffnungen 11 für die Spülflüssigkeit ausgebildet. Die Austrittsöffnungen 11 bilden das Ende von Kanälen 12, die in dem Aufsatz 5 ausgebildet sind und die an ihren den Austrittsöffnungen 11 abgewandten Enden an den Ringspalt 3 angeschlossen sind.

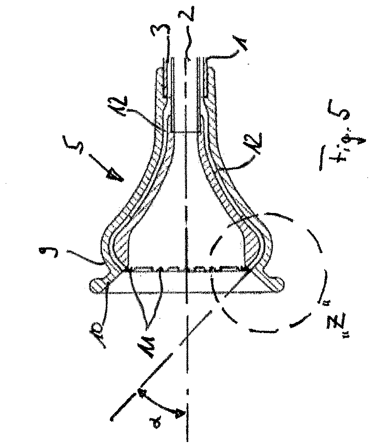

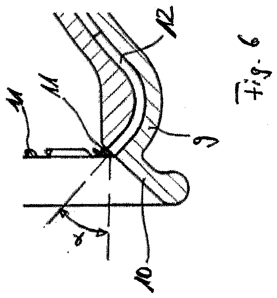

Die Austrittsöffnungen 11 sind erkennbar derart gestaltet, dass die Spülflüssigkeit unter einem Winkel α und somit schräg zur Längsmittellinie der Spülanordnung bzw. des Außenrohrs 1 sowie des Innenrohrs 2 austritt (vgl. Figuren 5 und 6). Schräg im Sinne der vorliegenden Erfindung bedeutet dabei, dass der Winkel α von der Längsmittellinie der Spülanordnung oder der rechtwinklig hierzu verlaufenden Linie in einem spitzen oder stumpfen Winkel abweicht. Der Winkel α beträgt dabei vorzugsweise zwischen 5° und 85° zur Längsmittellinie der Spülanordnung.

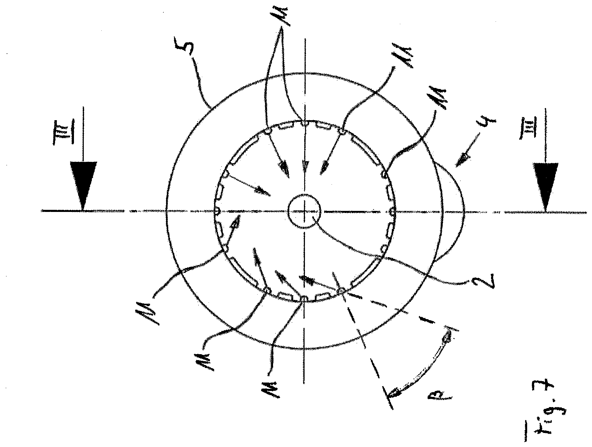

In der Darstellung gemäß Figur 7 sind zudem zwei verschiedene Ausgestaltungen des nicht erfindungsgemäßen Aufsatzes 5 mit der Einschnürung 9 dargestellt.

In der rechten Hälfte von Figur 7 tritt die Spülflüssigkeit derart aus den Austrittsöffnungen 11 aus, dass sie unter dem Winkel α in Richtung des Mittelpunkts der Spülanordnung gelenkt werden, wie dies durch die Pfeile angedeutet ist. Die Spülflüssigkeit trifft folglich im Zentrum der Wunde unter einem Winkel auf den zu spülenden Bereich.

In der linken Hälfte von Figur 7 tritt die Spülflüssigkeit ebenfalls unter dem Winkel α aus den Austrittsöffnungen 11 aus. Gleichzeitig tritt die Spülflüssigkeit darüber hinaus durch eine entsprechende Gestaltung der Austrittsöffnungen 11 unter einem Winkel β zur Radiallinie aus dem Aufsatz 5 aus, wie dies durch die Pfeile in der linken Hälfte von Figur 7 angedeutet ist. Die Spülflüssigkeit wird dadurch in eine Art Rotation versetzt, die eine Art Verwirbelung hervorruft, was zu einer weiteren Verbesserung des Spülergebnisses führt.

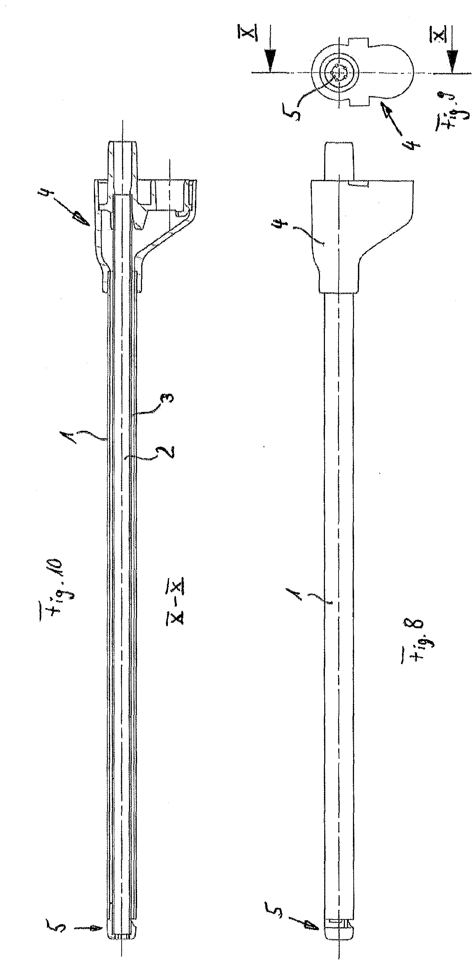

Im (nicht erfindungsgemäßen) Ausführungsbeispiel nach den Figuren 8 bis 11 ist der Aufsatz 5 kappenartig ausgebildet. Erkennbar ist der Aufsatz 5 außen abgerundet ausgeführt. Er ist mit Stegen 13 versehen, deren Wandstärke der Höhe des Ringspalts 3 entspricht. In montiertem Zustand sind die Stege 13 von dem Ringspalt 3 aufgenommen. In seinem Zentrum ist der Aufsatz 5 von einer Öffnung 14 durchsetzt, die koaxial zum Innenrohr 2 ausgerichtet ist, und deren Durchmesser im Wesentlichen dem Innendurchmesser des Innenrohres 2 entspricht. Durch die Öffnung 14 wird folglich die kontaminierte Spülflüssigkeit zum Innenrohr 2 gesaugt und durch dieses abgeführt.

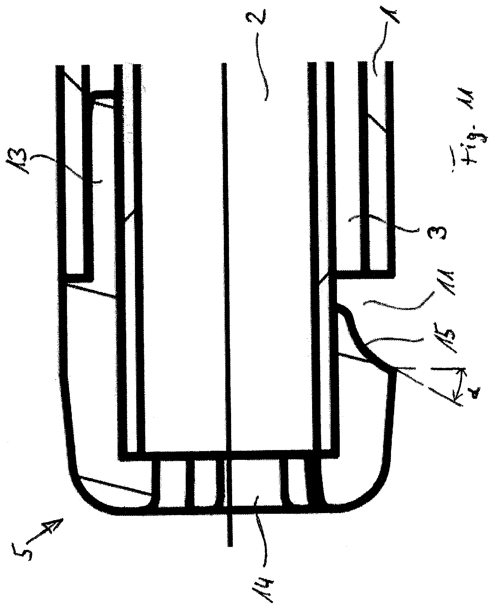

In dem Aufsatz 5 sind Austrittsöffnungen 11 für die Spülflüssigkeit ausgebildet. Die Austrittsöffnungen 11 sind in diesem Ausführungsbeispiel mit einer Prallfläche 15 versehen, die nach Art eines nach außen gerichteten Kreisabschnitts ausgeführt ist. Der Kreisabschnitt erstreckt sich im Ausführungsbeispiel über einen Bereich von etwa 25°. Erkennbar ist das äußerste Ende des Kreisabschnitts der Prallfläche 15 weder parallel noch rechtwinklig zur Längsmittellinie der Spülanordnung bzw. des Außenrohrs 1 sowie des Innenrohrs 2 ausgerichtet. Folglich tritt die Spülflüssigkeit schräg, nämlich unter dem Winkel α aus (vgl. Figur 11). Zudem ist es auch möglich, die Prallflächen 15 derart auszubilden, dass die Spülflüssigkeit die Austrittsöffnungen 11 unter dem Winkel β zur Radiallinie aus dem Aufsatz 5 austritt, wodurch auch bei diesem Ausführungsbeispiel eine Verbesserung der Spülwirkung erzielt werden kann.

In Abwandlung des Ausführungsbeispiels kann an der Öffnung 14 eine Art Kamm vorgesehen sein, die eine Filterfunktion erfüllen kann. Dadurch kann eine Verstopfung der Öffnung 14 bzw. des Innenrohrs 2 vermieden werden.

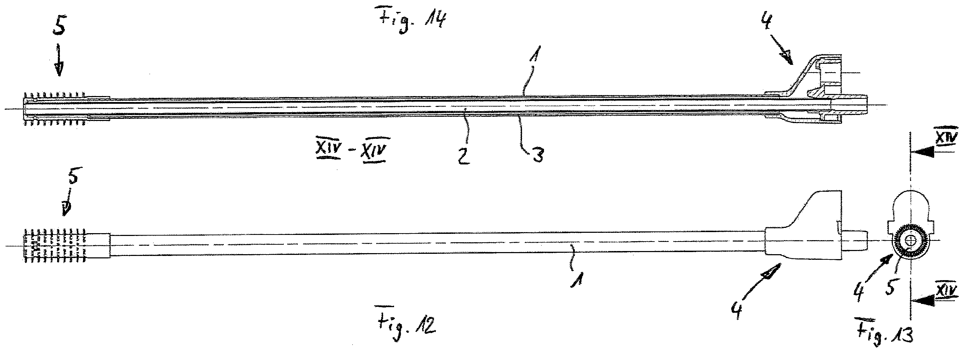

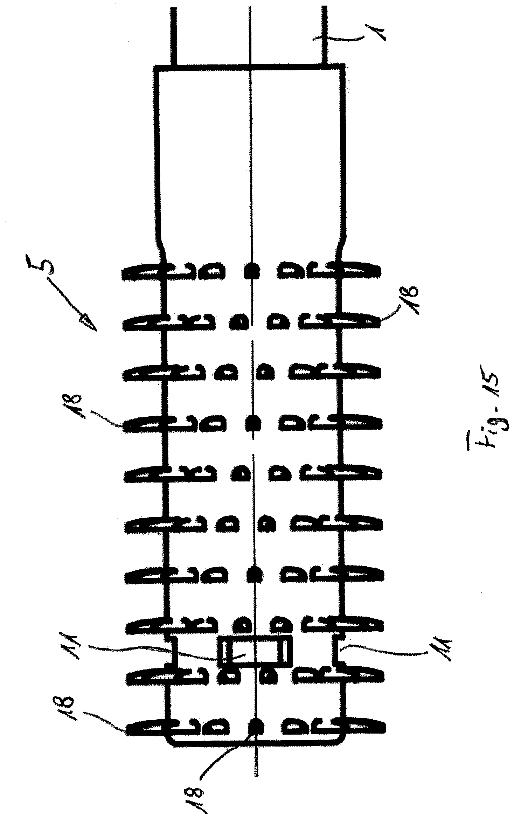

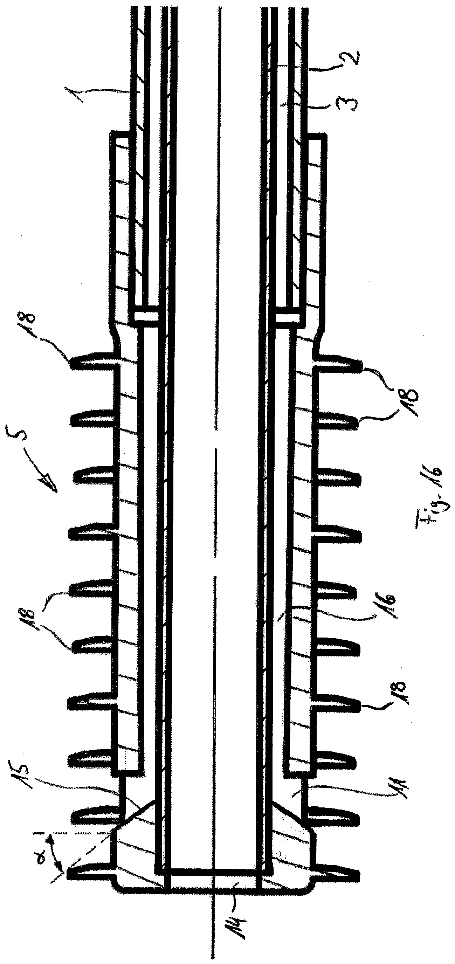

Im erfindungsgemäßen Ausführungsbeispiel nach den Figuren 12 bis 16 und 18 ist der Aufsatz 5 im Wesentlichen hohlzylindrisch ausgebildet. Er ist mit seinem dem Adapterstück 4 zugewandten Ende auf das Ende des Außenrohrs 1 aufgeschoben und umgibt den über das Außenrohr 1 hervorstehenden Bereich des Innenrohres 2. An seinem dem Adapterstück 4 abgewandten Ende stützt sich der Aufsatz 5 auf dem Innenrohr 2 ab. In diesem Bereich ist der Aufsatz 5 ebenfalls von einer Öffnung 14 durchsetzt. Der Durchmesser der Öffnung 14 entspricht dabei im Wesentlichen dem Innendurchmesser des Innenrohres 2. Durch die Öffnung 14 wird folglich auch bei diesem Ausführungsbeispiel die kontaminierte Spülflüssigkeit zum Innenrohr 2 gesaugt und durch dieses abgeführt.

Der Innendurchmesser des Aufsatzes 5 entspricht im Wesentlichen dem Innendurchmesser des Außenrohres 1. Folglich ist zwischen dem Innenrohr 2 und dem Aufsatz 5 ein Ringspalt 16 ausgebildet, der eine Fortsetzung des Ringspalts 3 bildet. Im Bereich des dem Adapterstück 4 abgewandten Endes ist der Aufsatz 5 mit Austrittsöffnungen 11 versehen, die nach Art von Fenstern ausgebildet sind. Im Ausführungsbeispiel sind vier Austrittsöffnungen 11 ausgebildet, die ringsum des Aufsatz 5 angeordnet sind. Eine andere Anzahl von Austrittsöffnungen 11 ist möglich. Auch können die Austrittsöffnungen 11 an anderen Orten des Aufsatzes 5 angeordnet sein. Ebenso ist es möglich, mehrere Ringe von Austrittsöffnungen 11 vorzusehen. In den Austrittsöffnungen 11 sind Prallflächen 15 vorgesehen, die weder parallel noch rechtwinklig zur Längsmittellinie der Spülanordnung bzw. des Außenrohrs 1 sowie des Innenrohrs 2 ausgerichtet sind. Folglich tritt die Spülflüssigkeit auch bei diesem Ausführungsbeispiel schräg, nämlich unter dem Winkel α aus dem Aufsatz 5 aus. Auch bei diesem Ausführungsbeispiel besteht die Möglichkeit, die Prallflächen 15 derart auszubilden, dass die Spülflüssigkeit die Austrittsöffnungen 11 unter dem Winkel β zur Radiallinie aus dem Aufsatz 5 austritt. Auf diese Weise kann auch bei diesem Ausführungsbeispiel die Spülflüssigkeit nach Art einer Rotation verwirbelt werden, was eine Verbesserung der Spülwirkung hervorruft.

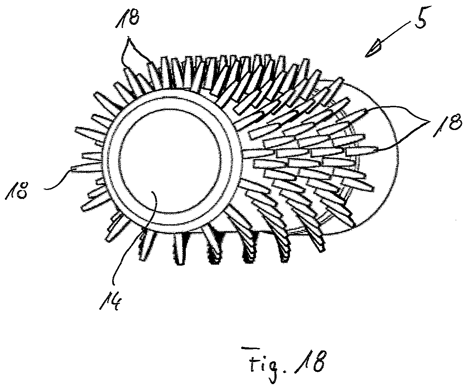

Auf seinem Umfang weist der Aufsatz 5 Borsten 18 auf. Die Borsten 18 sind aus demselben Material wie der Aufsatz. Sie sind an den Aufsatz 5 angeformt. Die Borsten 18 sind in Reihen nach Art einer Helix auf dem Umfang angeordnet, wie insbesondere Figur 18 zu entnehmen ist. Unter Helix, oder auch Schraube, Schraubenlinie, zylindrische Spirale oder Wendel genannt, ist eine Kurve zu verstehen, die sich mit konstanter Steigung um den Mantel eines Zylinders windet. Die Borstenreihen folgen somit einer Art Spirale um den Aufsatz 5. Sie weisen jeweils zwei scharfe Kanten 19 und eine Rundung 20 auf. Zudem sind die Borsten konisch ausgeführt. Die Anordnung der Borsten auf dem Umfang und die Anordnung der Austrittsöffnungen zwischen den Borsten ermöglicht den Austritt der Spülflüssigkeit zwischen den Borsten. Es wird daher dort gespült, wo auch gleichzeitig die mechanische Bearbeitung der Oberfläche erfolgt.

Durch die erfindungsgemäße Spülanordnung ist lediglich eine axiale Bewegung erforderlich ist, um alle Stellen der zu reinigenden Oberfläche zu erreichen. Da die Borsten nach Art einer Helix auf dem Umfang angeordnet sind, sind sie versetzt zueinander positioniert. Ihre Anordnung folgt einer Spirale um das Rohr. Durch diese Anordnung wird das Problem der zusätzlichen Rotationsbewegung beseitigt. Es ist nur ein axiales Einschieben der Spülanordnung erforderlich.

Durch die Anordnung der Borsten wird bei dem Einschieben jeder Bereich erreicht und mechanisch gebürstet.

## Patentansprüche

1. Spülanordnung für Spülsysteme zur Reinigung von Operationswunden mittels Spülflüssigkeit umfassend ein Außenrohr (1), in dem ein Innenrohr (2) angeordnet ist, wobei zwischen Außenrohr (1) und Innenrohr (2) mindestens abschnittsweise ein Ringspalt (3) ausgebildet ist, und an dem freien Ende des Außenrohrs (1) ein Aufsatz (5) vorgesehen ist, der auf seinem Umfang Borsten (18) aufweist, **dadurch gekennzeichnet, dass** die Borsten (18) nach Art einer Helix auf dem Umfang des Aufsatzes (5) angeordnet sind.

2. Spülanordnung nach Anspruch 1, **dadurch gekennzeichnet, dass** die Borsten (18) jeweils zwei scharfe Kanten (19) und eine Rundung (20) aufweisen.

3. Spülanordnung nach einem der vorgenannten Ansprüche, **dadurch gekennzeichnet, dass** die Borsten (18) konisch ausgeführt sind.

4. Spülanordnung nach einem der vorgenannten Ansprüche, **dadurch gekennzeichnet, dass** in dem Aufsatz (5) Austrittsöffnungen (11) für die Spülflüssigkeit ausgebildet sind, die derart gestaltet sind, dass die Spülflüssigkeit unter einem Winkel α und somit schräg zur Längsmittellinie der Rohre (1, 2) austritt.

5. Spülanordnung nach Anspruch 4, **dadurch gekennzeichnet, dass** der Winkel α zwischen 5° und 85° zur Längsmittellinie beträgt.

6. Spülanordnung nach Anspruch 4 oder 5, **dadurch gekennzeichnet, dass** der Aufsatz (5) das Innenrohr (2) abschnittsweise umgibt.

7. Spülanordnung nach einem der vorgenannten Ansprüche, **dadurch gekennzeichnet, dass** der Aufsatz (5) kappenartig ausgebildet ist.

8. Spülanordnung nach Anspruch 7, **dadurch gekennzeichnet, dass** der Aufsatz (5) außen abgerundet ausgeführt ist.

9. Spülanordnung nach einem der vorgenannten Ansprüche, **dadurch gekennzeichnet, dass** der Aufsatz (5) in seinem Zentrum von einer Öffnung (14) durchsetzt ist, die koaxial zum Innenrohr (2) ausgerichtet ist.

10. Spülanordnung nach Anspruch 4, **dadurch gekennzeichnet, dass** die Austrittsöffnungen (11) mit einer Prallfläche (15) versehen sind.

11. Spülanordnung nach Anspruch 10, **dadurch gekennzeichnet, dass** die Prallfläche (15) nach Art eines nach außen gerichteten Kreisabschnitts ausgeführt ist.

12. Spülanordnung nach einem der vorgenannten Ansprüche, **dadurch gekennzeichnet, dass** der Aufsatz (5) im Wesentlichen hohlzylindrisch ausgebildet ist.

13. Spülanordnung nach einem der vorgenannten Ansprüche, **dadurch gekennzeichnet, dass** der Innendurchmesser des Aufsatzes (5) im Wesentlichen dem Innendurchmesser des Außenrohres (1) entspricht.

14. Spülanordnung nach einem der vorgenannten Ansprüche, **dadurch gekennzeichnet, dass** ist zwischen dem Innenrohr (2) und dem Aufsatz (5) ein Ringspalt (16) ausgebildet ist.

15. Spülanordnung nach einem der vorgenannten Ansprüche, **dadurch gekennzeichnet, dass** die Austrittsöffnungen so gestaltet sind, dass die Spülflüssigkeit unter einem Winkel β zur Radiallinie aus den Austrittsöffnungen in dem Aufsatz (5) austritt.

## Claims

1. Flushing arrangement for flushing systems for cleaning surgical wounds by means of flushing liquid comprising an outer pipe (1), in which an inner pipe (2) is arranged, wherein between outer pipe (1) and inner pipe (2) an annular gap (3) is formed at least in sections, and on the free end of the outer pipe (1) an attachment (5) is provided, which has bristles (18) on its circumference, **characterised in that** the bristles (18) are arranged in the manner of a helix on the circumference of the attachment (5).

2. Flushing arrangement according to claim 1, **characterised in that** the bristles (18) each have two sharp edges (19) and a rounding (20).

3. Flushing arrangement according to one of the previous claims, **characterised in that** the bristles (18) are conical.

4. Flushing arrangement according to one of the previous claims, **characterised in that** outlet openings (11) for the flushing liquid are formed in the attachment (5), which are designed in such a manner that the flushing liquid exits at an angle α and thus obliquely to the longitudinal centre line of the pipes (1, 2).

5. Flushing arrangement according to claim 4, **characterised in that** the angle α is between 5° and 85° relative to the longitudinal centre line.

6. Flushing arrangement according to claim 4 or 5, **characterised in that** the attachment (5) sectionally surrounds the inner pipe (2).

7. Flushing arrangement according to one of the previous claims, **characterised in that** the attachment (5) is of cap-like design.

8. Flushing arrangement according to claim 7, **characterised in that** the attachment (5) is rounded on the outside.

9. Flushing arrangement according to one of the previous claims, **characterised in that** the attachment (5) has an opening (14) passing through its centre, which is coaxially aligned with the inner pipe (2).

10. Flushing arrangement according to claim 4, **characterised in that** the outlet openings (11) are provided with a baffle surface (15).

11. Flushing arrangement according to claim 10, **characterised in that** the baffle surface (15) is designed in the manner of an outwardly directed circular section.

12. Flushing arrangement according to one of the previous claims, **characterised in that** the attachment (5) is substantially hollow-cylindrical.

13. Flushing arrangement according one of the previous claims, **characterised in that** the inner diameter of the attachment (5) substantially corresponds to the inner diameter of the outer pipe (1).

14. Flushing arrangement according to one of the previous claims, **characterised in that** an annular gap (16) is formed between the inner pipe (2) and the attachment (5).

15. Flushing arrangement according to one of the previous claims, **characterised in that** the outlet openings are designed in such a manner that the flushing liquid exits the outlet openings in the attachment (5) at an angle β relative to the radial line.

## Revendications

1. Agencement de rinçage pour systèmes de rinçage destinés à nettoyer les plaies chirurgicales au moyen d'un liquide de rinçage, comprenant un tube extérieur (1) dans lequel est disposé un tube intérieur (2), sachant qu'entre le tube extérieur (1) et le tube intérieur (2) est configuré au moins de façon segmentée un interstice annulaire (3), et qu'à l'extrémité libre du tube extérieur (1) est prévu un embout (5), qui présente des crins (18) sur sa périphérie, **caractérisé en ce que** les crins (18) sont disposés de façon hélicoïdale sur la périphérie de l'embout (5).

2. Agencement de rinçage selon la revendication 1, **caractérisé en ce que** les crins (18) présentent respectivement deux arêtes vives et un arrondi.

3. Agencement de rinçage selon l'une des revendications précédentes, **caractérisé en ce que** les crins (18) sont réalisés coniques.

4. Agencement de rinçage selon la revendication 4, **caractérisé en ce que** dans l'embout (5) sont configurés des orifices de sortie (11) pour le liquide de rinçage, orifices dont la forme est telle que le liquide de rinçage sort selon un angle α et ainsi de biais par rapport à la ligne médiane longitudinale des tubes (1, 2).

5. Agencement de rinçage selon la revendication 4, **caractérisé en ce que** l'angle α est compris entre 5° et 85° par rapport à la ligne longitudinale médiane.

6. Agencement de rinçage selon la revendication 4 ou 5, **caractérisé en ce que** l'embout (5) entoure le tube intérieur (2) de façon segmentée.

7. Agencement de rinçage selon l'une des revendications précédentes, **caractérisé en ce que** l'embout (5) est configuré en forme de capuchon.

8. Agencement de rinçage selon la revendication 7, **caractérisé en ce que** l'embout (5) est réalisé arrondi à l'extérieur.

9. Agencement de rinçage selon l'une des revendications précédentes, **caractérisé en ce que** l'embout (5) est traversé en son centre par un orifice (14) en coaxialité avec le tube intérieur (2).

10. Agencement de rinçage selon la revendication 4, **caractérisé en ce que** les orifices de sortie (11) sont munis d'une chicane (15).

11. Agencement de rinçage selon la revendication 10, **caractérisé en ce que** la chicane (15) est réalisée à la façon d'un segment circulaire orienté vers l'extérieur.

12. Agencement de rinçage selon l'une des revendications précédentes, **caractérisé en ce que** l'embout (5) est configuré pour l'essentiel en forme cylindrique creuse.

13. Agencement de rinçage selon l'une des revendications précédentes, **caractérisé en ce que** le diamètre intérieur de l'embout (5) correspond pour l'essentiel au diamètre intérieur du tube extérieur (1).

14. Agencement de rinçage selon l'une des revendications précédentes, **caractérisé en ce qu'**entre le tube intérieur (2) et l'embout (5) est configuré un interstice annulaire (16).

15. Agencement de rinçage selon l'une des revendications précédentes, **caractérisé en ce que** les orifices de sortie sont configurés de sorte que le liquide de rinçage sort, selon un angle β par rapport à la ligne radiale, par les orifices de sortie ménagés dans l'embout (5).
